(19) Europäisches Patentamt
European Patent Office
Office européen des brevets

(11) EP 1 861 113 B1

(12) **FASCICULE DE BREVET EUROPEEN**

(45) Date de publication et mention de la délivrance du brevet:
**09.12.2009 Bulletin 2009/50**

(21) Numéro de dépôt: **06743787.1**

(22) Date de dépôt: **14.03.2006**

(51) Int Cl.:
***A61K 36/8962*** *(2006.01)* ***A23L 1/30*** *(2006.01)*
***A61K 31/70*** *(2006.01)* ***A61K 31/352*** *(2006.01)*
***A61P 3/00*** *(2006.01)*

(86) Numéro de dépôt international:
**PCT/FR2006/050224**

(87) Numéro de publication internationale:
**WO 2006/097660 (21.09.2006 Gazette 2006/38)**

(54) **UTILISATION D'UN EXTRAIT D'OIGNON POUR LA FABRICATION D'UNE COMPOSITION POUR CONTROLER LA PRISE DE POIDS**

VERWENDUNG EINES ZWIEBELEXTRAKTS ZUR HERSTELLUNG EINER ZUSAMMENSETZUNG ZUR KONTROLLE VON GEWICHTSZUNAHME

USE OF AN ONION EXTRACT FOR MAKING A COMPOSITION TO CONTROL WEIGHT GAIN

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **14.03.2005 FR 0550650**

(43) Date de publication de la demande:
**05.12.2007 Bulletin 2007/49**

(73) Titulaire: **Naturex**
**84140 Avignon (FR)**

(72) Inventeurs:
- **AMIOT-CARLIN, Marie-Josèphe**
  **F-84140 Montfavet (FR)**
- **JUHEL, Christine**
  **F-83270 Saint-cyr Sur Mer (FR)**
- **TOSINI, Frédéric**
  **F-83150 Bandol (FR)**
- **CARA, Louis,Résidence Petit Bosquet Bât A**
  **F-13012 Marseille (FR)**
- **BERGER, Véronique**
  **F-69003 Lyon (FR)**

(74) Mandataire: **Catherine, Alain et al**
**Cabinet Harlé & Phélip**
**7 rue de Madrid**
**75008 Paris (FR)**

(56) Documents cités:
**US-A1- 2002 187 207**

- **SALIM E I ET AL: "Lack of carcinogenicity of enzymatically modified isoquercitrin in F344/DuCrj rats" FOOD AND CHEMICAL TOXICOLOGY, XX, XX, vol. 42, no. 12, décembre 2004 (2004-12), pages 1949-1969, XP004610911 ISSN: 0278-6915**
- **MULLEN W ET AL: "Flavonoid metabolites in human plasma and urine after the consumption of red onions: analysis by liquid chromatography with photodiode array and full scan tandem mass spectrometric detection" JOURNAL OF CHROMATOGRAPHY, ELSEVIER SCIENCE PUBLISHERS B.V. AMSTERDAM, NL, vol. 1058, no. 1-2, 26 novembre 2004 (2004-11-26), pages 163-168, XP004638477 ISSN: 0021-9673**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 861 113 B1

## Description

La présente invention se rapporte au domaine des compositions nutritionnelles et des compositions pharmaceutiques destinées à réguler ou contrôler la prise de poids.

L'excès de poids corporel est un problème ayant une incidence croissante sur la santé humaine dans l'ensemble des pays industrialisés, ainsi que dans les pays en voie d'industrialisation rapide.

Aux Etats-Unis, environ 61% des individus adultes ont un excès de poids, qui est illustré avec un indice de masse corporelle (IMC) supérieur à 25 kilos par mètre carré. Les données de l' étude NHANES III qui datent de 1988 à 1994 montrent que 20 % des hommes et 25 % des femmes sont obèses (IMC > 30)

En France également, la fréquence des individus atteints d'un surpoids ou d'obésité croît dans les deux sexes. Des données publiées par l'AFSSA en juillet 2004 montrent que 19 % des enfants sont obèses. L'étude ObEpi/SOFRES de 2003 révèle que 11,3 % des adultes sont obèses (IMC > 30) et 41,6 % des adultes sont en surpoids (25 < IMC < 29,9). Le surpoids et l'obésité sont principalement dus au déséquilibre alimentaire et à l'insuffisance d'exercice physique et sont associés à de nombreuses maladies, tels que certains cancers, des maladies cardiovasculaires et le diabète.

Selon l'Organisation Mondiale de la Santé (OMS), l'excès pondéral et l'obésité sont les maladies chroniques les plus fréquentes rencontrées chez les enfants et les adolescents vivants dans les principaux pays industrialisés, y compris en Europe et aux Etats-Unis. Ainsi, selon l'Organisation Mondiale de la Santé, il existe dans le monde plus de 300.000 d'adultes obèses.

Les modifications environnementales et comportementales apportées par le développement économique, la modernisation et l'urbanisation sont associées à l'accroissement global de l'obésité chez les adultes et les enfants.

L'accroissement du poids corporel résulte d'un déséquilibre entre les apports et les dépenses d'énergies chez l'individu, et se manifeste par une expansion excessive de la masse de tissu adipeux. L'excès de poids, y compris l'obésité, accroît le risque d'hypertension, de diabète de type II, d'arthrite, de niveau élevé d'hyperlipidémie, de cancer, de grossesse à risque ou encore d'asthme.

Les approches diététiques pour la régulation de l'excès de poids corporel ont eu un succès limité. Les régimes à basses calories peuvent provoquer une perte de poids temporaire, mais ils n'ont pas apporté la preuve qu'ils pouvaient constituer des solutions à long terme pour les personnes recherchant une perte de poids, puis le maintien d'un poids réduit.

Les médicaments qui suppriment l'appétit, qui réduisent la prise alimentaire, qui augmentent les dépenses d'énergie et/ou qui modifient le métabolisme ont une efficacité potentielle pour réduire la prise de poids. Malheureusement, ces médicaments sont fréquemment associés à divers effets indésirables, certains d'entre eux pouvant être, dans de rares cas, mortels.

Il existe donc un besoin dans l'état de la technique pour de nouvelles compositions permettant le contrôle de la prise de poids, qui soient efficaces, qui possèdent des effets indésirables réduits, et qui soient d'un prix de revient faible.

De telles compositions permettant le contrôle de la prise de poids chez l'homme ou l'animal sont fournies par la présente invention. Dans la pratique, les compositions de l'invention sont utilisées par des individus qui observent préférentiellement simultanément un contrôler de la prise alimentaire.

De manière surprenante, on a montré selon la présente invention qu'un extrait purifié d'oignon possédant une forte teneur en composés polyphénoliques possède une grande efficacité dans la limitation de la prise pondérale d'un mammifère soumis à un régime athérogène, riche en lipides.

La présente invention a pour objet l'utilisation d'un extrait d'oignon comprenant de 20% à 40% en poids de polyphénols de la famille des flavonols, ledit extrait d'oignon ayant les caractéristiques de composition suivantes, exprimées en poids, par rapport au poids total de l'extrait sec :

- 60% à 90% en poids des flavonols totaux dudit extrait consistent en des flavonols glycosylés,
- 85% à 98% en poids des flavonols totaux dudit extrait consistent en de la quercétine sous forme libre (aglycone) ou glycosylée,
- 5% à 20% en poids des flavonols totaux dudit extrait consistent en la quercétine-3,4'-diglucoside,
- 30% à 70% en poids des flavonols totaux dudit extrait consistent en un mélange de quercétine-3-monoglucoside et de quercétine-4'-monoglucoside, et
- 20 à 30% en poids des flavonols totaux dudit extrait consistent en la quercétine,
pour la fabrication d'une composition pour contrôler la prise de poids.

Par « quercétine » et « glycosides de la quercétine », on entend selon l'invention les composés ayant la formule (I) suivante:

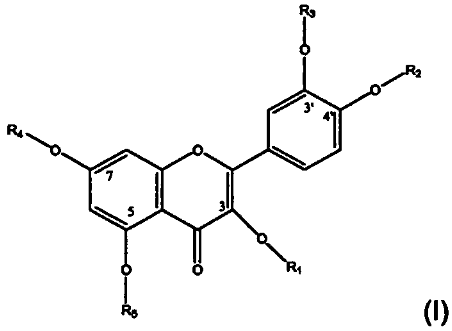

(I)

dans laquelle :

les groupes $R_1$ à $R_5$ signifient, indépendamment les uns des autres, un résidu osidique ou une chaîne polyosidique possédant de 1 à 3 unités d'oside, ou un atome d'hydrogène.

Le composé de quercétine sous forme libre (aglycone) est un composé de formule (I), dans laquelle les groupes $R_1$ à $R_5$ signifient chacun un atome d'hydrogène.

Le composé quercétine-3,4'-diglucoside est le composé de formule (I) dans laquelle les groupes $R_1$ et $R_2$ signifient chacun un résidu osidique de glucose et les groupes $R_3$, $R_4$ et $R_5$ signifient chacun un atome d'hydrogène.

Le composé de quercétine-3-monoglucoside est le composé de formule (I) dans laquelle le groupe $R_1$ signifie un résidu osidique de glucose et les groupes $R_2$, $R_3$ et $R_4$ signifient chacun un atome d'hydrogène.

Le composé de quercétine-4'-monoglucoside est le composé de formule (I) dans laquelle le groupe $R_2$ signifie un résidu osidique de glucose et les groupes $R_1$, $R_3$, $R_4$ et $R_5$ signifient chacun un atome d'hydrogène.

Parmi les composés glycosides de la quercétine susceptibles d'être contenus dans l'extrait d'oignon défini ci-dessus, on peut citer notamment la quercitrine, qui est un composé de formule (I) dans laquelle le groupe $R_1$ signifie un résidu osidique de 6-déoxy-$\alpha$-L-mannopyranosyl, et les groupes $R_2$, $R_3$, $R_4$ et $R_5$ signifient un atome d'hydrogène.

Parmi les composés susceptibles d'être contenus dans l'extrait d'oignon défini ci-dessus, on peut aussi citer la rutine, qui est un composé de formule (I) dans laquelle le groupe $R_1$ signifie un groupe rutinose, et les groupes $R_2$, $R_3$, $R_4$, et $R_5$ signifient un atome d'hydrogène. Comme cela est connu, le groupe rutinose est un disaccharide formé de deux résidus osidiques, respectivement le rhamnose (encore appelé 6-déoxy-L-mannose) et de glucose.

Comme exemples de glycosides de quercétine susceptibles d'être contenus dans l'extrait d'oignon défini ci-dessus, on peut citer la quercétine-3-O-$\beta$-D-gtucosyl-(1$\rightarrow$2)-$\beta$-D-glucosyl-(1$\rightarrow$2)-beta-D-glucosyde, dans laquelle le groupe $R_1$ signifie le groupe $\beta$-D-glucosyl-(1$\rightarrow$2)-bêta-D-glucoside et les groupes $R_2$, $R_3$, $R_4$ et $R_5$ signifient un atome d'hydrogène.

On peut citer également les glycosides de la quercétine qui consistent en des composés de formule (I) dans lesquels l'un des groupes $R_1$, $R_2$, $R_3$, $R_4$ et $R_5$, mais plus spécifiquement le groupe $R_1$, signifie l'un des groupes osidiques choisis parmi $\alpha$-L-(5"-O-acétyl)-arabinofuranose, $\alpha$-L-arabinofuranose, $\alpha$-L-(3"-O-acétyl)-arabinofuranose, ou $\alpha$-L-rhamnopyranose.

Un autre flavanol est le composé de isorhamnétine, qui est un composé de formule (I), dans laquelle le groupe $R_3$ signifie le groupe méthyl ($CH_3$) et les groupes $R_1$, $R_2$, $R_4$ et $R_5$ signifient chacun un atome d'hydrogène.

Un autre flavanol est le composé quercétine-3,4'-isorhamnétine, qui est le composé de formule (I) dans laquelle le groupe $R_3$ signifie le groupe méthyl ($CH_3$), les groupes $R_1$ et $R_2$ signifient chacun un résidu osidique de glucose et les groupes $R_4$ et $R_5$ signifient chacun un atome d'hydrogène.

Un autre flavanol est le composé de isorhamnétine-4'-monoglucoside, qui est le composé de formule (I) dans laquelle le groupe $R_3$ signifie le groupe méthyl ($CH_3$), le groupe $R_2$ signifie un résidu osidique de glucose et les groupes $R_1$, $R_4$ et $R_5$ signifient chacun un atome d'hydrogène.

Le kaempférol est un autre flavonol susceptbile d'être contenu dans l'extrait d'oignon défini ci-dessus, en général à une teneur inférieure à 1 % en poids, par rapport au poids total de l'extrait sec.

Par « kaempférol », on entend selon l'invention le composé ayant la formule (II) suivante:

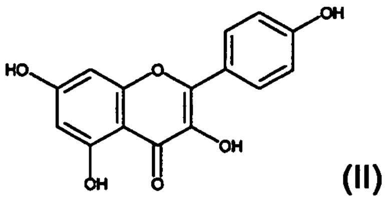

(II)

De manière générale, pour les caractéristiques de composition de l'extrait d'oignon, les teneurs sont exprimées en équivalent quercétine-3'monoglucoside ou isoquercitrine, pour les flavonols glycosylés, ou en équivalent quercétine pour la quercétine et les autres flavonols aglycones.
On définit par « équivalent » l'utilisation d'un standard externe pour la quantification en estimant que le facteur de réponse du composé à doser est comparable au facteur de réponse du standard.

Ainsi, on a montré selon l'invention qu'un extrait purifié d'oignon riche en polyphénols de la famille des flavonols, dont 85% à 98% en poids sec des flavonols consiste en une quercétine ou des glycosides de quercétine, possède une activité de limitation de la prise de poids chez des mammifères soumis à un régime alimentaire dit « athérogène » riche en lipides,.

De manière tout aussi surprenante, on a montré selon l'invention que l'extrait d'oignon purifié riche en polyphénols, notamment en quercétine et glycosides de quercétine, tel que défini ci-dessus, possédait une activité de limitation de la prise de poids du même ordre de grandeur que la tétrahydrolipstatine ([ 2S-[2-$\alpha$(R), 3$\beta$]]-N-formyl-L-leucine 1-[(3-hexyl-4-oxo-2-oxetanyl) méthyl] dodécyl ester ). La tétrahydrolipstatine est un principe actif de grande efficacité utilisé pour le traitement de l'obésité en association avec un régime hypocalorique, et pour le traitement du diabète non insulino-dépendant.

La tétrahydrolypstatine est un principe actif qui inhibe spécifiquement la lipase pancréatique, ce qui a pour effet de réduire l'absorption des lipides alimentaires d'environ un tiers en moyenne, chez l'homme, ce qui induit une perte de poids.

Toutefois, la tétrahydrolipstatine est associée à certains effets secondaires, tels que la stéatorrhée, le suintement anal, des douleurs abdominales, des nausées, des vomissements ou encore une diminution de l'absorption des vitamines liposolubles.

De plus, on a aussi montré selon l'invention que l'extrait purifié d'oignon riche en polyphénols, en particulier en quercétine et glycosides de quercétine, défini ci-dessus, possède une activité de limitation de la prise de poids bien supérieure à un autre extrait végétal riche en polyphénols, plus spécifiquement un extrait de marc de raisin riche en polyphénols. On sait que les polyphénols totaux du raisin permettent une diminution de 9,7% de la cholestérolémie après douze semaines de régime athérogène (Auger C, Gerain P, Laurent-Bichon F, Portet K, Bomet A, Caporiccio B, Croc G, Teissedre PL, Rouanet JM ; 2004, J. Agric. Food Chem.K, vol.52(16): 5297-5302).

Selon un premier aspect préféré, dans l'extrait d'oignon purifié défini ci-dessus, de 70% à 80% en poids des polyphénols totaux de l'extrait sec consistent en des flavonols glycosylés.

Selon un second aspect préféré, de 90% à 95% en poids des flavonols dudit extrait sec consistent en une quercétine ou des glycosides de quercétine.

Selon un troisième aspect préféré, de 7% à 13% en poids des flavonols totaux dudit extrait sec consistent en la quercétine-3,4'-diglucoside.

Selon un quatrième aspect préféré, de 40% à 60% en poids des flavonols totaux dudit extrait sec consistent en de quercétine-3-monoglucoside et de quercétine-4'-monoglycoside.

Selon un cinquième aspect préféré, ledit extrait d'oignon purifié comprend de 25% à 35% en poids de l'extrait sec de polyphénols de la famille des flavonols.

L'analyse du contenu en polyphénols, en particulier en flavonols, d'un extrait d'oignon purifié tel que défini ci-dessus peut être réalisé par l'homme du métier selon toute technique séparative appropriée connue en soi. Par exemple, l'homme du métier peut réaliser l'analyse du contenu en polyphénols d'un extrait purifié sec d'oignon tel que défini ci-dessus selon diverses techniques d'analyse parmi lesquelles des techniques de chromatographie décrites par Baranowski et al. (Baranowski R, Kabut J., Baranowska I, 2004, Analytical Letters, vol. 37 (1):157-165), de Degenhardt et al. ( Degenhardt A, Engelhardt UH, Lackenbrink C, Winterhalter P, 2000, J. Agric. Food Chem., vol. 48 (8) : 3425-3430), de Crozier et al. (Crozier A, Burns J., Aziz A.A, Stewart A J Rabiasz HS, Jenkins GI, Edwards CA and Lean MEJ, Biol. Res. , Vol.33 (2): 79-88) et de Guillen et al. (Guillen DA, Barroso CG, Perez-Bustramante J A , 1996, Journal of Chromatography A; viol.724 (1): 117-124). L'homme du métier peut également utiliser les techniques de séparation des polyphénols par électrophorèse capillaire, comme décrit notamment par Pazourek et al. (Pazourek J., Gonzalez G, Revilla AL, Havel J, 2000, Journal of Chromatography A., vol. 874 (1): 111-119).

L'analyse du contenu en polyphénols d'un extrait d'oignon tel que défini ci-dessus peut également être réalisée

selon le procédé suivant :

Les composés phénoliques sont séparés par HPLC sur une colonne Alltima 5 μm (150 x 4.6 mm, Alltech) protégée par colonne de garde C18 Alltima à l'aide d'un gradient composé de deux solvants A ($H_2O$ + HCOOH 0.05%), B ($CH_3CN$), à 0 min , 10% B ; à 40 min, 40% B ; à 50 min, 100% B . Le débit est de 1 mL.$min^{-1}$ et la séparation s'effectue à 35°C. Le volume injecté est de 20 μl. La détection est réalisée par spectrophotométrie à 365 nm. Les composés sont quantifiés par étalonnage externe. Les teneurs sont exprimées en équivalent isoquercitrine ou quercétine-3'-monoglucoside (QMG) pour les glycosides de quercétine ou en équivalent quercétine pour les autres flavonols aglycones.

De manière tout à fait préférée, l'extrait sec purifié d'oignon tel que défini ci-dessus se présente, en tant que composant de base d'une composition selon l'invention, sous la forme d'une poudre.

De manière tout à fait préférée, l'extrait purifié sec d'oignon riche en polyphénols de la famille des flavonols ci-dessus est obtenu par la mise en oeuvre d'un procédé d'extraction, de fractionnement et de purification des composés polyphénoliques issus d'oignons comprenant les étapes suivantes :

a) extraction des composés polyphénoliques pour obtenir un extrait brut d'oignon;
b) adsorption sur une résine adsorbante des composés polyphénoliques contenus dans l'extrait brut ;
c) élution des composés polyphénoliques retenus sur la résine pour obtenir un extrait purifié ;
d) concentration, puis séchage éventuel, de l'extrait purifié pour obtenir un produit riche en composés polyphénoliques.

De manière tout à fait préférée, l'étape b) d'adsorption est réalisée sur une résine de type styrène-divinylbenzène, et encore mieux une résine de ce type présentant les caractéristiques physiques suivantes :

1) des pores ayant une taille moyenne allant de 50 à 110 Angströms, de préférence de 60 à 100 Angströms,
2) une surface spécifique égale ou supérieure à 800 $m^2$/g, de préférence égale ou supérieure à 880 $m^2$/g,
3) un volume de pores supérieur à 1 ml/g, et de préférence égal ou supérieur à 1,4 ml/g.

De manière tout à fait préférée, l'étape a) d'extraction comprend une étape de chauffage rapide de la matière végétale d'oignon, par exemple les écarts de triage d'oignons, depuis une température ambiante de 25°C jusqu'à une température de 105°C afin d'obtenir un jus d'exsudation chaud et un mou d'oignon cuit exsudé refroidi, le jus d'exsudation constituant au moins partiellement l'extrait brut, puis une étape de mise sous vide de la matière végétale d'oignon, provoquant la vaporisation d'une partie de celle-ci, cette étape étant réalisée après l'étape de chauffage, à une pression comprise entre $10^3$ et 2 x $10^4$ Pa absolus.

De manière tout à fait préférée, l'étape b) d'adsorption est réalisée avec une résine de type styrène-divinylbenzène choisie parmi les résines commercialisées par la Société Resindion sous les dénominations SP70 et SP700.

Un procédé particulièrement préféré d'obtention de l'extrait sec purifié d'oignon utilisé selon l'invention est le procédé décrit dans la demande PCT N°WO 02/064536, au contenu complet duquel l'homme du métier se référera avantageusement.

Selon un aspect particulièrement préféré de l'invention, on utilise pour fabriquer une composition pour contrôler la prise de poids, exclusivement un extrait sec purifié d'oignon tel que défini ci-dessus comme matière physiologiquement active sur la prise de poids.

Notamment, selon cet aspect préféré de l'invention, on n'utilise, pour fabriquer une composition pour contrôler la prise de poids, aucune matière végétale additionnelle, autre que l'extrait purifié sec d'oignon défini dans la présente description.

En particulier, dans un de ses aspects préférés, la composition finale pour contrôler la prise de poids qui est fabriquée ne contient pas de composés additionnels permettant la réadsorption (« re-uptake») de neurotransmetteurs tels que la norépinéphrine, la sérotonine ou la dopamine.

De même, selon cet aspect préféré de l'invention, la composition finale pour contrôler la prise de poids qui est fabriquée ne contient pas de composés additionnels ayant une action sur l'absorption et la synthèse des graisses, du cholestérol et des triglycérides, comme par exemple l'acide hydroxycitrique.

En particulier, selon cet aspect préféré de l'invention, la composition finale pour contrôler la prise de poids qui est fabriquée ne contient aucun composé additionnel de type polyphénol, en particulier de type flavonol, et encore plus particulièrement de type quercétine ou glycosides de la quercétine, autres que ceux qui sont déjà contenus dans l'extrait sec purifié d'oignon qui est défini dans la présente description.

Selon un premier mode de réalisation, l'extrait sec purifié d'oignon défini ci-dessus est utilisé pour la fabrication d'une composition nutritionnelle ou alimentaire destinée à contrôler la prise de poids.

Selon un second mode de réalisation, l'extrait sec purifié d'oignon défini ci-dessus est utilisé pour la fabrication d'une composition pharmaceutique destinée à contrôler la prise de poids.

***Compositions nutritionnelles de l'invention***

De préférence, une composition nutritionnelle qui est fabriquée selon l'invention comprend une quantité d'extrait sec purifié d'oignon adaptée à une administration orale quotidienne dudit extrait d'oignon comprise entre 0,1 g et 10 g.

Pour une consommation chez l'homme, une composition nutritionnelle selon l'invention comprend une quantité d'extrait sec purifié d'oignon définie ci-dessus adaptée à un apport quotidien en ledit extrait, fourni par ladite composition, comprise entre 0,7 g à 5 g et de manière tout à fait préférée de 1,5 g à 2 g.

Pour une consommation par un animal, spécifiquement un mammifère non humain, y compris le chien, le chat ou le cheval, une composition nutritionnelle selon l'invention est adaptée à une administration quotidienne de l'extrait sec purifié d'oignon, fournie par ladite composition, comprise entre 1 et 10 g, de préférence entre 1 et 5 g dudit extrait.

Selon encore un autre aspect, la composition nutritionnelle ci-dessus peut comprendre d'autres composés nutritionnels, en combinaison avec l'extrait sec purifié d'oignon.

Ainsi, une composition nutritionnelle selon l'invention peut également comprendre une source de calcium, par exemple sous la forme d'un composé organique ou inorganique physiologiquement acceptable, tels que des sels de calcium inorganiques (chlorure de calcium, phosphate de calcium, sulfate de calcium, oxyde de calcium, hydroxyde de calcium ou carbonate de calcium) ou des composants organiques contenant du calcium telle que la poudre de lait écrémé, le caséinate de calcium ou encore des sels organiques de calcium (citrate de calcium, maléate de calcium ou leurs mélanges).

Une composition nutritionnelle selon l'invention peut également comprendre des vitamines, telles que la vitamine A, la vitamine D, la vitamine E, la vitamine K, la vitamine C, l'acide folique, la thiamine, la riboflavine, la vitamine $B_6$, la vitamine $B_{12}$, la niacine, la biotine ou encore l'acide pantothénique.

Une composition nutritionnelle selon l'invention peut également comprendre des éléments minéraux et des éléments traces tels que le sodium, le potassium, le phosphore, le magnésium, le cuivre, le zinc, le fer, le sélénium, le chrome et le molybdène.

Elle peut également comprendre des fibres solubles telles que l'agar-agar, un alginate, du caroube, du carraghenane, de la gomme arabique, de la gomme de guar, de la gomme de karaya, de la pectine ou de la gomme xanthane, ces fibres solubles étant sous une forme hydrolysée ou non hydrolysée.

Elle peut aussi comprendre des composés source d'énergie, en des quantités faibles de manière à ne pas induire un régime alimentaire hypercalorique, notamment une ou plusieurs sources d'hydrate de carbone choisie parmi les maltodextrines, l'amidon, le lactose, le glucose, le sucrose, le fructose, le xylitol et le sorbitol.

En outre, une composition nutritionnelle selon l'invention peut également comprendre des arômes naturels ou artificiels, par exemple des arômes de fruits comme la banane, l'orange, la pêche, l'ananas ou la framboise, ou d'autres arômes végétaux comme la vanille, le cacao, le café, etc.

Une composition nutritionnelle selon l'invention peut se présenter sous la forme d'une poudre. Elle peut également se présenter sous la forme de gélules en gélatine dure contenant une telle poudre, sous la forme de comprimés ou encore de concentré liquide ou sirop. Elle peut également être incorporée à un aliment de consommation courante tels que les produits frais 4ème gamme comme des salades prêt-à-l'emploi ; les produits $5^{ème}$ gamme ; les purées, plats cuisinés ; soupes et potages ; jus à base de légumes (tomate, carottes ...) ; produits laitiers (fromage frais ...) ; les produits d'assaisonnements : vinaigre, vinaigrette ou mélange d'aromates.

Comme cela a déjà été mentionné précédemment, une composition destinée à contrôler la prise de poids selon l'invention peut aussi être représentée sous la forme d'une composition pharmaceutique, comme cela est décrit ci-dessous.

***Compositions pharmaceutiques***

Il peut s'agir d'une composition pharmaceutique humaine ou vétérinaire, en particulier pour le chien ou le chat, ou encore le cheval.

Une composition pharmaceutique destinée à contrôler la prise de poids qui est fabriquée à partir d'un extrait purifié sec d'oignon, comme défini ci-dessus, se présente sous une forme pour l'administration orale, parentérale ou intra-veineuse. Les formes orales sont particulièrement préférées.

Dans sa forme destinée à l'administration humaine, une composition pharmaceutique fabriquée selon l'invention comprend avantageusement une quantité d'extrait sec purifiée d'oignon adaptée pour une administration quotidienne dudit extrait sec purifié d'oignon, fournie par ladite composition, comprise entre 0,1 g et 10 g d'extrait sec purifié d'oignon.

Sous sa forme destinée à une administration chez l'animal, une composition pharmaceutique fabriquée selon l'invention comprend une quantité d'extrait sec purifiée d'oignon adaptée à l'administration quotidienne dudit extrait, fournie par ladite composition, comprise entre 0,5 g et 10 g.

Une composition pharmaceutique telle que définie ci-dessus comprend l'extrait sec purifié d'oignon en association avec au moins un excipient choisi dans le groupe constitué par les excipients pharmaceutiquement acceptables.

Des techniques de préparation de compositions pharmaceutiques peuvent être aisément retrouvées par l'homme du métier, par exemple dans l'ouvrage Remmington's Pharmaceutical Sciences, Mid. Publis. Co, Easton, PA, USA.
Des adjuvants, des véhicules et des excipients physiologiquement acceptables sont aussi décrits dans l'ouvrage intitulé "Handbook of Pharmaceutical Excipients, seconde édition, American Pharmaceutical Association , 1994".
Pour formuler une composition pharmaceutique selon l'invention, l'homme du métier pourra avantageusement se référer à la dernière édition de la Pharmacopée Européenne ou de la Pharmacopée des Etats-Unis d'Amérique (USP).
L'homme du métier pourra notamment avantageusement se référer à la quatrième édition « 2002 » de la Pharmacopée Européenne, ou encore à l'édition USP 25-NF 20 de la Pharmacopée Américaine (U.S. Pharmacopeia).
L'invention concerne aussi une méthode pour contrôler la prise de poids d'un patient, ladite méthode comprenant une étape au cours de laquelle on administre aux patients, une quantité thérapeutiquement efficace d'un extrait sec purifié d'oignon tel que défini dans la présente description, ou encore d'une composition pharmaceutique telle que définie ci-dessus.
Une composition pharmaceutique fabriquée selon l'invention se présente sous une forme liquide, ou , de manière préférentielle, sous une forme solide.
Pour une administration orale, on préférera une composition pharmaceutique solide, par exemple sous la forme de comprimés, de capsules ou de gélules.
Sous la forme liquide, on préférera une composition pharmaceutique sous la forme d'une suspension aqueuse.
Des formes pharmaceutiques solides peuvent comprendre, en tant que véhicules, adjuvants ou excipients, au moins un agent diluant, un arôme, un agent solubilisant, un agent lubrifiant, un agent de suspension, un agent désintégrant et un agent d'encapsulation, l'identité et la fonction de ces différents classiques étant documentés complètement dans la Pharmacopée Européenne ou dans la Pharmacopée des Etats-Unis d'Amérique, (USP).
De tels composés sont par exemple le carbonate de magnésium, le stéarate de magnésium, le talc, le lactose, la pectine, la dextrine, l'amidon, la gélatine, des matériaux cellulosiques, etc.
Les compositions sous forme liquide peuvent comprendre également de l'eau, le cas échéant en mélange avec du propylèneglycol ou du polyéthylèneglycol, et éventuellement aussi des agents de coloration, des arômes, des stabilisants et des agents épaississants.
L'invention est en outre illustrée, sans pour autant être limitée, par la figure et les exemples suivants.
La figure 1 illustre les résultats de l'évolution pondérale des hamsters soumis à différents régimes : STD (régime athérogène standard), EVO-1 (0,06 % d'extrait d'oignon), EVO-2 (0,10% d'extrait d'oignon), EVO-3 (0,15% d'extrait d'oignon), XEN (0,03 % de Xénical®), OPC (0,15% de Mincigrap®).

## EXEMPLES

### *A. MA TERIEL ET METHODES*

### *A.1. Choix des quantités de produits à tester*

Le seul médicament « inhibiteur de lipase digestive » commercialement disponible sur ordonnance médicale est le Xénical® (Laboratoire Roche). Son principe actif, la « tétrahydrolipstatine » inhibe spécifiquement la lipase pancréatique, ce qui réduit l'absorption des lipides alimentaires d'environ un tiers en moyenne, chez l'Homme (Zhi et *al.* 1994), et aide ainsi à perdre du poids (Sjöstrôm et *al.* 1998). De nombreux compléments alimentaires, produits de phytothérapie, sont aussi proposés pour initier et/ou aider l'amaigrissement.
Dans la présente étude, les effets de l'extrait d'oignon EV06/05/02 ont été comparés à ceux du produit commercialisé sous la marque « Mincigrap® » par la Société Laboratoires Arkopharma. En effet, le produit « Mincigrap® » constitue une source importante de polyphénols de marc de raisin dont les effets sur les enzymes digestives ont été démontrés *in vitro* (Griffiths 1986, Tebib *et al.* 1994, Carmona 1996) et *in vivo* chez le rat (Horigome et al. 1988, Vallet *et al.* 1994).
On recommande aux personnes en surpoids (IMC $\geq$ 25 kg/$m^2$) ou obèses (IMC $\geq$ 30 kg/$m^2$) qui souhaitent maigrir de réduire leur apport calorique de façon plus ou moins prononcée (1400 à 2200 kCal/j) dans le cadre d'une alimentation équilibrée (apportant 45 à 75 g/j de lipides). C'est à partir de ces données, présentées dans le Tableau 1, ci-après, que les doses de Xénical®, de « Mincigrap® » et d'extrait d'oignon EV06/05/02 à administrer aux hamsters ont été déterminées.

**Tableau 1 : Posologie et quantités de Xénical® ou de « Mincigrap® » rapportées au poids corporel et aux lipides alimentaires ingérés chez l'Homme.**

| Spécialité | Posologie | Dose en mg/kg PC (60-120 kg) | Dose en mg/g TG (45-75 g de lipides) |
|---|---|---|---|
| Xénical® | 3 gélules de 240 mg 720 mg/j | 6 à 12 | 10 à 16 |
| « Mincigrap® » | 6 gélules de 390 mg 2340 mg/j | 20 à 40 | 31 à 52 |

Puisque l'étude est réalisée chez un modèle animal type rongeur non-obèse, mais qui reçoit un régime enrichi en lipides, on a testé les doses de produits suivantes :

- pour le Xénical® : 12,5 mg/kg de poids corporel,
- pour « Mincigrap® » : 50 mg/kg de poids corporel.

Les quantités d'extrait d'oignon EV06/05/02 selon l'invention sont choisies, d'une part, de manière à ce qu'elles soient légèrement supérieures à celle du Xénical®, qui est un médicament et non un complément alimentaire, et d'autre part, de manière à ce qu'elles permettent d'établir une comparaison avec le produit « Mincigrap® », qui est une source de polyphénols :

- $1^{ère}$ dose testée : 30 mg/kg de poids corporel,
- $2^{ème}$ dose testée : 50 mg/kg de poids corporel,
- $3^{ème}$ dose testée : 75 mg/kg de poids corporel.

***A.2. Animaux et régimes***

Le hamster doré, de souche Aura Rj, est choisi comme modèle animal (Élevage Janvier).

Des hamsters mâles, pesant entre 83 et 113 grammes au départ de l'étude, sont placés en cages de 4 au maximum, dans une salle climatisée à 22°C. Après 10 jours d'adaptation à l'animalerie, les hamsters sont répartis en 6 groupes et sont nourris pendant 14 jours avec soit une alimentation standard pauvre en lipides (groupe témoin), soit un régime « test », c'est-à-dire un régime athérogène contenant soit l'extrait d'oignon, soit le Xénical®, soit le produit « Mincigrap® » :

- Groupe témoin ou « STD » (n = 8) : aliment d'entretien pour hamsters
- Groupe EV0-1 (n = 8) : régime athérogène contenant 0,06 % d'extrait EV06/05/02.
- Groupe EV0-2 (n = 8) : régime athérogène contenant 0,10 % d'extrait EV06/05/02.
- Groupe EV0-3 (n = 8) : régime athérogène contenant 0,15 % d'extrait EV06/05/02.
- Groupe XEN (n = 8) : régime athérogène contenant 0,03 % de Xénical®.
- Groupe MCG (n = 8) : régime athérogène contenant 0,15 % de « Mincigrap® ».

Le régime contrôle est un régime d'entretien pour hamsters UAR04 (usine d'alimentation rationnelle, Villemoisson-sur-Orge, France), de valeur énergétique (3400 kcal/kg).

L'athérogénicité des régimes « tests » est obtenue par un enrichissement en cholestérol (0,25 %) et en lipides (10 %) qui sont apportés par de l'huile d'olive (1/2), de l'huile de tournesol (1/4) et du suif (1/4). Les régimes « tests » sont isoprotéiques, isoglucidiques et isolipidiques et ont une valeur énergétique de 3620 kcal/kg. Ces régimes « tests » ont été fabriqués par Mucedola SRL (Settimo Milanese, Italie) d'après la formulation représentée dans le Tableau 2 ci-dessous.

**Tableau 2** : Compositions des régimes « tests »

| Ingrédients g/100 g de régime | EV0-1 | EV0-2 | EV0-3 | XEN | MCG |
|---|---|---|---|---|---|
| EV06/05/02 | 0,06 | 0,10 | 0,15 | - | - |
| Xénical® | - | - | - | 0,03 | - |
| « Mincigrap® » | - | - | - | - | 0,10 |

(suite)

| Ingrédients g/100 g de régime | EV0-1 | EV0-2 | EV0-3 | XEN | MCG |
|---|---|---|---|---|---|
| Cholestérol | 0,25 | 0,25 | 0,25 | 0,25 | 0,25 |
| Lipides | 10,0 | 10,0 | 10,0 | 10,0 | 10,0 |
| Saccharose | 20,00 | 20,00 | 20,00 | 20,00 | 20,00 |
| Amidon | 24,00 | 24,00 | 24,00 | 24,00 | 24,00 |
| Cellulose | 5,00 | 5,00 | 5,00 | 5,00 | 5,00 |
| Protéines | 24,00 | 24,00 | 24,00 | 24,00 | 24,00 |
| Vitamines | 1,25 | 1,25 | 1,25 | 1,25 | 1,25 |
| Minéraux | 5,00 | 5,00 | 5,00 | 5,00 | 5,00 |
| Eau | 10,44 | 10,40 | 10,35 | 10,47 | 10,40 |

### *A.3. Traitement des données*

Le traitement statistique des données est effectué à l'aide du logiciel Stat-View V, commercialisé par la Société SAS Institute Inc., Cary NC, USA.

Les résultats (poids et variations de poids, ingérés, pourcentages d'absorption, lipémies et tests d'oxydabilité des lipoprotéines) sont exprimés par la moyenne ± SEM (erreur standard à la moyenne) et sont tous présentés en annexe (paragraphe 6).

Pour un paramètre donné, les différences statistiquement significatives entre les valeurs moyennes des différents groupes sont déterminées par l'analyse de la variance (ANOVA) pour mesures non répétées à la probabilité de P<0,05.

Le coefficient d'absorption est calculé comme suit :

$$\text{\% absorption} = \left(1 - \frac{{}^{14}\text{C total dans les fèces}/{}^{3}\text{H total dans les fèces}}{{}^{14}\text{C total dans le bolus}/{}^{3}\text{H total dans le bolus}}\right) \times 100$$

### A.4. Analyse qualitative et quantitative de l'extrait sec d'oignon.

Une analyse qualitative et quantitative d'un extrait sec d'oignon selon l'invention est détaillée dans le Tableau ci-dessous.

| ***Molécules*** | **Polyphénols** (g/100g d'extrait sec) |
|---|---|
| Quercétine-3,4'-diglucoside | 1,63 |
| Isorhamnétine-3,4'-diglucoside | 0,41 |
| Autres Quercétines diglucosides | 1,60 |
| Quercétine-3-monoglucoside | 0,57 |
| Quercétine-4'-monoglucoside | 18,89 |
| Isorhamnétine-4'-monoglucoside | 0,21 |
| Quercétine | 9,99 |
| Isorhamnétine | 0,19 |
| Kaempférol | 0,13 |
| **Flavonols totaux** | **33,62** |

**B. EXEMPLE 1 : Utilisation d'une composition comprenant un extrait purifié d'oignon riche en flavonols pour contrôler la prise de poids.**

### *B.1. Évolution pondérale et Ingesta*

#### *B.1.1. Évolution pondérale*

Les hamsters de chaque groupe ont été pesés en début (J1) et en fin d'expérimentation (J14). Les moyennes des poids à J1 et à J14 sont présentées dans le Tableau 3, ci-après. Les variations de poids entre J1 et J14 sont présentées dans la figure 1.

**Tableau 3 : Moyenne des poids (grammes) des hamsters de chaque groupe à J1 et J14.**

| Groupes | STD | EV0-1 | EV0-2 | EV0-3 | XEN | MCG |
|---|---|---|---|---|---|---|
| **Poids à J1 (g)** | 92,9 ± 2,1 | 102,5 ± 1,7 | 101,8 ± 1,6 | 104.6 ± 1.5 | 105,3 ± 1,6 | 103,4 ± 2,3 |
| **Poids à J14 (g)** | 101,3 ± 1,9 | 104,3 ± 1,9 | 103,4 ± 1,6 | 106,0 ± 1,4 | 106,5 ± 2,5 | 108,0 ± 2,3 |
| *STD versus EV0-1, EV0-2, EV0-3, XEN (P < 0,0001), MCG (P < 0,01) MCG versus EV0-1, EV0-2, EV0-3, XEN (P < 0,05)* | | | | | | |

Par comparaison aux 5 groupes tests, le groupe STD prend significativement plus de poids (8,4 ± 0,5 g versus 1,25 ± 1,49 g pour le groupe XEN et 4,62 ± 1,03 g pour le groupe MCG). Les hamsters des 3 groupes EV0 et du groupe XEN ont significativement moins pris de poids que ceux du groupe MCG (2,6 à 3,3 fois).
Enfin, aucun effet-dose de l'extrait EV06/05/02 sur la prise pondérale n'est mis en évidence.

Les données bibliographiques confirment que l'évolution pondérale du groupe STD est conforme à celle de ce modèle animal (il est donc possible de d'établir des commentaires référencés sur les effets des produits à tester sur ce paramètre).

Plusieurs études d'interventions nutritionnelles menées chez le hamster doré recevant une alimentation riche en lipides (de 12 à 20 % selon les études) permettent d'évaluer les effets de tels régimes sur la prise pondérale. Ces effets sont présentés dans le Tableau 4, ci-après :

**Tableau 4 : Moyenne des poids (grammes) des hamsters de chaque groupe à J1 et J14.**

| Références | Quantité et nature des lipides | Prise de poids (g/j) |
|---|---|---|
| Kurowka & Manthey (2004) | 12 % de lipides apportés par de l'huile de noix de coco (10 %) et de l'huile de carthame (2 %) et 0,1 % de cholestérol | 0,60 ± 0,22 et 0,62 ± 0,24 |
| Auger et al. (2004) | 15 % de lipides, apportés sous forme de saindoux, et 0,5 % de cholestérol | Estimée de 0,38 à 0,63 selon le poids initial des hamsters |
| Nicolisi et al. (1998) | 20 % de lipides sources d'acides gras monoinsaturés (acide acide oléique) et saturés (acide myristique) | 0,71 (acide oléique) et 0,68 (acide myristique) |
| Résultats de la présente étude | 10 % de lipides (mélange de sources) et 0,25 % de cholestérol | 0,13 (EV0-1), 0,11 (EVO-2), 0,10 (EV0-3), 0,09 (XEN) et 0,33 (MCG). |

L'analyse de ces données est intéressante puisqu'elle révèle que les hamsters nourris avec les régimes contenant l'extrait d'oignon EV06/05/02, le Xénical® ou le « Mincigrap® » ont une prise de poids (g/j) moyenne très faible compte tenu de l'enrichissement en lipides de ces régimes (10 % versus 4 % pour le régime d'entretien).

Toutefois, dans les études de Auger et *al.* (2004), Kurowka & Manthey (2004), ainsi que dans l'étude de Wiseman *et al.* (2002), l'enrichissement de ces régimes en polyphénols (poudre de polyphénols de pépins et de marc de raisin, polyphénols d'agrumes, et polyphénols hydrosolubles d'olive, respectivement) sont sans effet sur la prise de poids. Ces résultats suggèrent que les effets sur la prise de poids observés dans cette étude pourraient être spécifiques à l'extrait d'oignon, et probablement aux flavonols (quercétine, isorhamnétine et kaempférol) qu'il contient. Chez l'Homme, comme chez l'animal, peu de données mettant en relation la prise de polyphénols, à l'exception des polyphénols de thé, c'est à dire essentiellement les catéchines, et le maintien ou la perte de poids sont disponibles. Toutefois, il n'existe aucune donnée rapportant un effet des flavonols, en particulier de la quercétine ou de glycosides de la quercétine sur

la prise de poids.

Enfin, concernant les effets du Xénical®, une étude réalisée chez le rat rendu obèse puis recevant le Xénical® (54 mg/kg de poids corporel) montre que la perte de poids de ces rats est de 8 % (Hogan *et al.* 1987). Dans une autre étude menée chez le rat non-obèse, l'utilisation de 2 mg de Xénical® par gramme de lipides (3 mg/g de lipides dans la présente étude), diminue la prise de poids de 81 % (Ackroff & Sclafani, 1996). Ce résultat semble plus en accord avec l'effet du Xénical® sur l'évolution pondérale qui est démontré dans la présente étude : la variation de poids chez les hamsters traités au Xénical® par comparaison aux hamsters non traités (groupe STD) est de 85 %.

### *B.1.2. Ingesta et doses de produits à tester*

Les quantités moyennes d'aliments ingérés par chaque hamster par jour ont été mesurées par pesée des granulés distribués et résiduels en début et en fin d'intervention nutritionnelle. Ces données permettent de calculer l'énergie moyenne ingérée par jour ainsi que les doses d'extrait d'oignon, de Xénical® (XEN) ou de « Mincigrap® » (MCG) réellement administrées (voir Tableau 5 ci-dessous).

Tableau 5 : Énergies totales ingérées (kcal par kg de poids corporel et par j) et doses de produits à tester réellement administrées (mg par kg de poids corporel).

| | STD | EV0-1 | EV0-2 | EV0-3 | XEN | MCG |
|---|---|---|---|---|---|---|
| **Énergie totale (kCal/kg.j)** | 191,8 ± 4,0 | 164,7 ± 2,7 | 168,2 ±2,6 | 165,9 ± 2,3 | 186,5 ± 2,7 | 184,8 ±4,1 |
| **Doses de produit test (mg/kg PC)** | - | EV06/05/02 27,0 ± 0,5 | EV06/05/02 46,0 ± 0,8 | EV06/05/02 69,0 ± 0,9 | Xénical® 15,4 ± 0,3 | «Mincigrap®» 49,8 ± 1,0 |

Les hamsters des groupes XEN et MCG ont une consommation énergétique comparable à ceux du groupe STD alors que les hamsters des groupes EV0 ont une consommation inférieure (-14 % à -12 % pour les groupes EV0-1 et EV0-2, respectivement). De la même façon, l'ingéré du groupe XEN est identique à celui du groupe MCG (<1 %) mais reste significativement supérieur à celui des groupes EV0-1 (11,7 %), EV0-2 (11,0 %) et EV0-3 (9,8 %).

Par comparaison aux données bibliographiques, les quantités ingérées (en moyenne 4,96 $\pm$ 0,15 g/j) par les hamsters de cette étude sont en accord avec celles d'études menées dans des conditions expérimentales similaires, c'est-à-dire utilisant soit une alimentation d'entretien pour hamster (Gilat et *al.* 2003), soit une alimentation enrichie en lipides (voir références du Tableau 5). Dans ce dernier cas, les ingérés varient de 4,60 $\pm$0,10 g/j (Auger et *al.* 2004) à 7,6$\pm$0,8 g/j (Kurwska & Manthey 2004) et 9,8 $\pm$ 0,3 g/j (Nicolosi *et al.* 1998).

***<u>Conclusion du paragraphe B.1. :</u>***

L'extrait d'oignon EV06/05/02 limite la prise de poids de façon significative par comparaison à un régime standard et par comparaison à la poudre de marc de raisin « Mincigrap® », utilisée à la même dose. L'efficacité est maximale dès la plus faible dose (27 mg/kg PC) et comparable à celle du Xénical® (15 mg/kg PC).

Contrairement aux résultats d'investigation des effets du Xénical® menée chez l'animal, aucune étude ne rapporte d'effet limitant de la prise de poids par l'ingestion chronique de polyphénols.

***B.2. Conclusion***

***B.2.1. Effets de l'extrait EV06/05/02 par comparaison au régime standard***

L'extrait EV06/05/02 agit de façon remarquable sur la prise de poids par comparaison au régime standard. Ainsi, les hamsters prennent 6,7 fois, 4,8 fois et 5,4 fois moins de poids lorsqu'ils reçoivent une alimentation supplémentée avec l'extrait EV06/05/02 aux doses 27, 46 et 69 mg/kg de poids corporel, respectivement.

On peut noter toutefois que, si les hamsters nourris avec les régimes contenant l'extrait EV06/05/02 ont un apport calorique rapporté à leur poids corporel inférieur à ceux du groupe standard, cette différence reste minime (12 à 14 %) et ne peut être responsable de la perte de poids.

Les résultats de l'étude confirment que l'extrait d'oignon EV06/05/02 diminue l'assimilation des triglycérides alimentaires *in vivo* et montrent les effets favorables de cet extrait sur la prise pondérale. Si aucun effet/dose n'est démontré clairement, il est important de souligner l'efficacité de cet extrait avec une dose minimale (27 mg/kg de poids corporel ou 6 mg/g de triglycérides).

***B.2.2. Extrait EV06/05/02 versus Xénical®***

L'effet sur la prise de poids de l'extrait EV06/05/02 est comparable à celui du Xénical®. Ces résultats montrent clairement que l'extrait EV06/05/02 utilisé à des doses de 27 mg/kg PC (soit seulement 2 fois celle du Xénical®) est aussi efficace que le Xénical® pour limiter la prise pondérale.

Ces données suggèrent que l'action de l'extrait EV06/05/02 sur la prise pondérale ne serait pas, ou dans tous les cas pas uniquement, liée à l'inhibition de l'assimilation des lipides et que d'autres mécanismes, propres aux composants de l'extrait d'oignon, seraient impliqués. Cette hypothèse est intéressante si on considère qu'une inhibition trop importante de la digestion des lipides, telle que celle induite par le Xénical®, présente des effets secondaires sur le confort des sujets (nausées, vomissements, diarrhées, douleurs abdominales etc ...), et à plus long terme, a des effets délétères sur le statut vitaminique.

L'extrait d'oignon EV06/05/02, quelle que soit la dose testée, présente une efficacité comparable à celle du Xénical® sur la prise de poids même si l'absorption des triglycérides est moins altérée.

***B.2.3. Extrait EV06/05/02 versus « Mincigrap® »***

En dépit d'un effet moins marqué sur l'absorption des lipides alimentaires, l'extrait d'oignon EV06/05/02 limite plus efficacement et significativement la prise de poids que le « Mincigrap® » : d'un facteur 3 à dose équivalente (46 mg/kg PC pour EV06/05/02 ; 49 mg/kg PC pour « Mincigrap® »), d'un facteur 2,6 fois pour la dose 27 mg/kg PC et d'un facteur 3,4 fois pour la dose 69 mg/kg PC.

Par comparaison à «Mincigrap® », l'efficacité d'EV6/05/02, quelle que soit la dose, sur la prise de poids est nettement supérieure, alors que l'excrétion fécale des lipides alimentaires est plus faible.

Les résultats de cette étude montrent que l'extrait d'oignon agit sur la prise de poids. Bien que les résultats ne soient pas présentés ici, on a montré que l'extrait d'oignon selon l'invention agit sur la prise de poids, sans induire une inhibition drastique de l'absorption des triglycérides chez le hamster. Cette observation est intéressante dans la mesure

où un effet modéré sur la digestion des lipides (et probablement des autres macronutriments) devrait atténuer les perturbations de la biodisponibilité des micronutriments (vitamines liposolubles et hydrosolubles, minéraux et oligo-éléments), et par conséquent les limiter les risques de déficiences.

Puisque l'efficacité sur l'évolution pondérale est comparable à celle du Xénical® et supérieure à celle du « Mincigrap® », qui induisent une excrétion fécale plus importante des lipides alimentaires, il est permis de penser que l'extrait d'oignon agit sur ce paramètre par d'autres mécanismes qu'il conviendrait d'explorer.

La comparaison des effets de l'extrait d'oignon sur la prise de poids avec le Xénical® et le « Mincigrap® » permet, toutefois, de comparer l'activité de cet extrait par rapport à un inhibiteur spécifique des lipases, dont les effets ont été validés chez l'Homme, et par rapport à un inhibiteur non spécifique des enzymes digestives.

En conclusion, les résultats de l'exemple 1 montrent que l'extrait d'oignon EV06/05/02 est actif sur la perte de poids à une dose raisonnable d'un point de vue nutritionnel et physiologique chez le hamster (27 à 69 mg/kg de PC), ce qui correspondrait à une quantité ingérée par jour chez l'Homme de 1,5 à 2 grammes. Une telle propriété justifierait l'utilisation de l'extrait d'oignon comme une « aide à la perte de poids » chez l'Homme.

## REFERENCES

Zhi J, Melia AT, Guerciolini R, Chung J, Kinberg J, Hauptman JB, Patel IH. Rétrospective population-based analysis of the dose-response (fecal fat excrétion) relationship of orlistat in normal and obese volunteers. Clin Pharmacol Ther 1994 Jul;56(1):82-5

Sjöström L, Rissanen A, Andersen T, Boldrin M, Golay A, Koppeschaar HP, Krempf M. (1998). Randomised placebo-controlled trial of orlistat for weight loss and prevention of weight regain in obese patients. European Multicentre Orlistat Study Group. Lancet Jul 18;352(9123):167-72.

Griffiths DW. The inhibition of digestive enzymes by polyphenolic compounds. Adv Exp Med Biol. 1986;199:509-16.

Carmona A. Tannins: thermostable pigments which complex dietary proteins and inhibit digestive enzymes. Arch Latinoam Nutr. 1996 Dec;44(4 Suppl 1):31S-35S.

Tebib K, Rouanet JM, Besancon P. Effect of grape seed tannins on the activity of some rat intestinal enzyme activities. Enzyme Protein. 1994-95;48(1):51-60.

Auger C, Gerain P, Laurent-Bichon F, Portet K, Bomet A, Caporiccio B, Cros G, Teissedre PL, Rouanet JM. Phenolics from commercialized grape extracts prevent early atherosclerotic lesions in hamsters by mechanisms other than antioxidant effect. J Agric Food Chem. 2004 Aug 11;52(16):5297-302.

Kurowska EM, Manthey JA. Hypolipidemic effects and absorption of citrus polymethoxylated flavones in hamsters with diet-induced hypercholesterolemia. J Agric Food Chem. 2004 May 19;52(10):2879-86.

Wiseman SA, Tijburg LB, van de Put FH. Olive oil phenolics protect LDL and spare vitamin E in the hamster. Lipids. 2002 Nov;37(11):1053-7.

Gilat T, Leikin-Frenkel A, Goldiner I, Juhel C, Lafont H, Gobbi D, Konikoff FM. Prevention of diet-induced fatty liver in experimental animals by the oral administration of a fatty acid bile acid conjugate (FABAC). Hepatology. 2003 Aug;38(2):436-42.

Nicolosi RJ, Wilson TA, Rogers EJ, Kritchevsky D. Effects of specific fatty acids (8:0, 14:0, cis-18:1, trans-18:1) on plasma lipoproteins, early atherogenic potential, and LDL oxidative properties in the hamster. J Lipid Res. 1998 Oct; 39(10):1972-80.


## Revendications

1. Utilisation d'un extrait d'oignon comprenant de 20% à 40% en poids de polyphénols de la famille des flavonols, ledit extrait d'oignon ayant les caractéristiques de composition suivantes, exprimées en poids par rapport au poids total de l'extrait sec :

- 60% à 90% en poids des flavonols totaux dudit extrait consistent en des polyphénols glycosylés,
- 85% à 98% en poids des flavonols totaux dudit extrait consistent en la quercétine sous forme libre (aglycone) ou glycosylée,
- 5% à 20% en poids des flavonols totaux dudit extrait consistent en la quercétine-3,4'-diglucoside,
- 30% à 70% en poids des flavonols totaux dudit extrait consistent en de la quercétine-3-monoglucoside et de la quercétine-4'-monoglucoside, et
- 20 à 30% en poids des flavonols totaux dudit extrait consistent en la quercétine,

pour la fabrication d'une composition pour contrôler la prise de poids.

**2.** Utilisation selon la revendication 1, **caractérisée en ce que,** dans ledit extrait d'oignon, 70% à 80% en poids des polyphénols totaux dudit extrait consistent en des flavonols glycosylés.

**3.** Utilisation selon l'une des revendications 1 et 2, **caractérisée en ce que,** dans ledit extrait d'oignon, 90% à 95% en poids des flavonols totaux dudit extrait consistent en une quercétine sous forme libre (aglycone) ou glycosylée.

**4.** Utilisation selon l'une des revendications 1 à 3, **caractérisée en ce que,** dans ledit extrait d'oignon, 7% à 13% en poids des flavonols totaux dudit extrait consistent en la quercétine-3,4'-diglucoside.

**5.** Utilisation selon l'une des revendications 1 à 4, **caractérisée en ce que,** dans ledit extrait d'oignon, 40% à 60% en poids des flavonols totaux dudit extrait consistent en la quercétine-3-monoglucoside et la quercétine-4'-monoglucoside.

**6.** Utilisation selon l'une des revendications 1 à 5, **caractérisée en ce que** ledit extrait d'oignon comprend de 25% à 35% en poids de polyphénols de la famille des flavonols.

**7.** Utilisation selon l'une des revendications 1 à 6, pour la fabrication d'une composition nutritionnelle ou alimentaire pour contrôler la prise de poids.

**8.** Utilisation selon l'une des revendications 1 à 6, pour la fabrication d'une composition pharmaceutique pour contrôler la prise de poids.

## Claims

**1.** Use of an onion extract comprising from 20% to 40% by weight of polyphenols belonging to the flavonol family, said onion extract having following composition characteristics, expressed in weight based on the dry extract total weight:

- from 60% to 90% by weight of the total flavonols in said extract consist in glycosylated polyphenols,
- from 85% to 98% by weight of the total flavonols in said extract consist in quercetin in a free form (aglycone) or in a glycosylated form,
- from 5% to 20% by weight of the total flavonols in said extract consist in quercetin-3,4'-diglucoside,
- from 30% to 70% by weight of the total flavonols in said extract consist in quercetin-3-monoglucoside and quercetin-4'-monoglucoside, and
- from 20 to 30% by weight of the total flavonols in said extract consist in quercetin,

for preparing a weight gain control composition.

**2.** Use according to claim 1, **characterized in that**, in said onion extract, from 70% to 80% by weight of total polyphenols in said extract consist in glycosylated flavonols.

**3.** Use according to any of the claims 1 and 2, **characterized in that**, in said onion extract, from 90% to 95% by weight of the total flavonols in said extract represent a quercetin in a free form (aglycone) or in a glycosylated form.

**4.** Use according to any of the claims 1 to 3, **characterized in that**, in said onion extract, from 7% to 13% by weight of the total flavonols in said extract consist in quercetin-3,4'-diglucoside.

**5.** Use according to any of the claims 1 to 4, **characterized in that**, in said onion extract, from 40% to 60% by weight of the total flavonols in said extract consist in quercetin-3-monoglucoside and quercetin-4'-monoglucoside.

6. Use according to any of the claims 1 to 5, **characterized in that** said onion extract comprises from 25% to 35% by weight of polyphenols belonging to the flavonol family.

7. Use according to any of the claims 1 to 6, for preparing a nutritional or a food composition for controlling the weight gain.

8. Use according to any of the claims 1 to 6, for preparing a pharmaceutical composition for controlling the weight gain.

**Patentansprüche**

1. Verwendung eines Zwiebelextrakts, der 20 bis 40 Gew.-% Polyphenole aus der Familie der Flavonole umfasst, wobei der Zwiebelextrakt die folgenden Zusammensetzungsmerkmale hat, ausgedrückt in Gewicht pro Gesamtgewicht des Trockenextrakts:

   - 60 bis 90 Gew.-% der Gesamtflavonole des Extrakts bestehen aus glycosylierten Polyphenolen;
   - 85 bis 98 Gew.-% der Gesamtflavonole des Extrakts bestehen aus Quercetin in freier (Aglycon) oder glycosylierter Form;
   - 5 bis 20 Gew.-% der Gesamtflavonole des Extrakts bestehen aus Quercetin-3,4'-diglucosid;
   - 30 bis 70 Gew.-% der Gesamtflavonole des Extrakts bestehen aus Quercetin-3-monoglucosid und Quercetin-4'-monoglucosid; und
   - 20 bis 30 Gew.-% der Gesamtflavonole des Extrakts bestehen aus Quercetin;

   zur Herstellung einer Zusammensetzung zur Einschränkung der Gewichtszunahme.

2. Verwendung gemäß Anspruch 1, **dadurch gekennzeichnet, dass** in dem Zwiebelextrakt 70 bis 80 Gew.-% der Gesamtpolyphenole des Extrakts aus glycosylierten Flavonolen bestehen.

3. Verwendung gemäß einem der Ansprüche 1 und 2, **dadurch gekennzeichnet, dass** in dem Zwiebelextrakt 90 bis 95 Gew.-% der Gesamtflavonole des Extrakts aus einem Quercetin in freier (Aglycon) oder glycosylierter Form bestehen.

4. Verwendung gemäß einem der Ansprüche 1 bis 3, **dadurch gekennzeichnet, dass** in dem Zwiebelextrakt 7 bis 13 Gew.-% der Gesamtflavonole des Extrakts aus Quercetin-3,4'-diglucosid bestehen.

5. Verwendung gemäß einem der Ansprüche 1 bis 4, **dadurch gekennzeichnet, dass** in dem Zwiebelextrakt 40 bis 60 Gew.-% der Gesamtflavonole des Extrakts aus Quercetin-3-monoglucosid und Quercetin-4'-monoglucosid bestehen.

6. Verwendung gemäß einem der Ansprüche 1 bis 5, **dadurch gekennzeichnet, dass** der Zwiebelextrakt 25 bis 35 Gew.-% Polyphenole aus der Familie der Flavonole umfasst.

7. Verwendung gemäß einem der Ansprüche 1 bis 6 zur Herstellung einer Nähr- oder Nahrungszusammensetzung zur Einschränkung der Gewichtszunahme.

8. Verwendung gemäß einem der Ansprüche 1 bis 6 zur Herstellung einer pharmazeutischen Zusammensetzung zur Einschränkung der Gewichtszunahme.

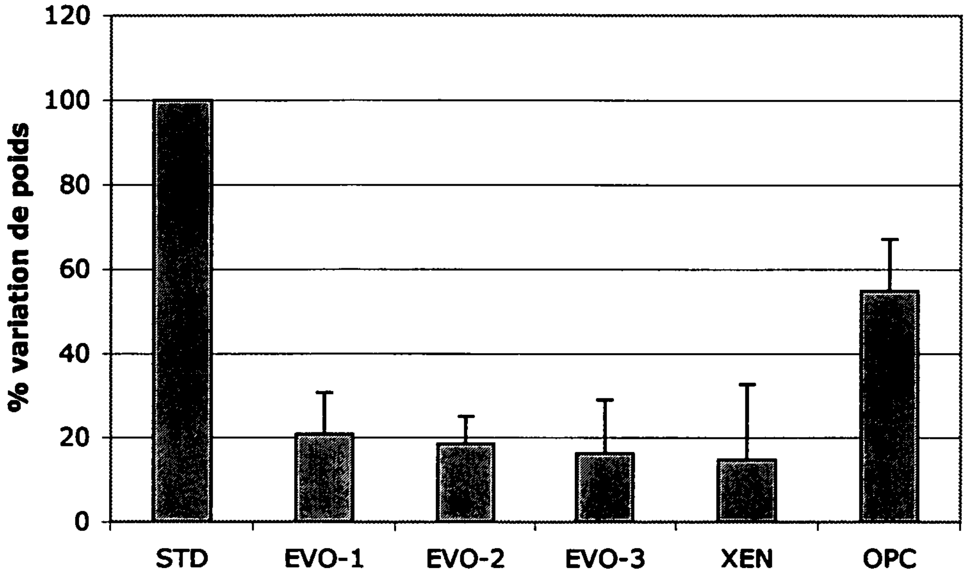
120
100
80
60
40
20
0
% variation de poids
STD
EVO-1
EVO-2
EVO-3
XEN
OPC

FIGURE 1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 02064536 A


### Littérature non-brevet citée dans la description


- **Auger C ; Gerain P ; Laurent-Bichon F ; Portet K ; Bomet A ; Caporiccio B ; Croc G ; Teissedre PL ; Rouanet JM.** *J. Agric. Food Chem.K,* 2004, vol. 52 (16), 5297-5302
- **Baranowski R ; Kabut J. ; Baranowska I.** *Analytical Letters,* 2004, vol. 37 (1), 157-165
- **Degenhardt A ; Engelhardt UH ; Lackenbrink C ; Winterhalter P.** *J. Agric. Food Chem.,* 2000, vol. 48 (8), 3425-3430
- **Crozier A ; Burns J. ; Aziz A.A ; Stewart A J ; Rabiasz HS ; Jenkins GI ; Edwards CA ; Lean MEJ.** *Biol. Res.,* vol. 33 (2), 79-88
- **Guillen DA ; Barroso CG ; Perez-Bustramante J A.** *Journal of Chromatography A,* 1996, vol. 724 (1), 117-124
- **Pazourek J. ; Gonzalez G ; Revilla AL ; Havel J.** *Journal of Chromatography A.,* 2000, vol. 874 (1), 111-119
- Handbook of Pharmaceutical Excipients. American Pharmaceutical Association, 1994
- **Zhi J ; Melia AT ; Guerciolini R ; Chung J ; Kinberg J ; Hauptman JB.** Patel IH. Rétrospective population-based analysis of the dose-response (fecal fat excrétion) relationship of orlistat in normal and obese volunteers. *Clin Pharmacol Ther,* Juillet 1994, vol. 56 (1), 82-5
- **Sjöström L ; Rissanen A ; Andersen T ; Boldrin M ; Golay A ; Koppeschaar HP ; Krempf M.** Randomised placebo-controlled trial of orlistat for weight loss and prevention of weight regain in obese patients. *European Multicentre Orlistat Study Group. Lancet,* 18 Juillet 1998, vol. 352 (9123), 167-72
- **Griffiths DW.** The inhibition of digestive enzymes by polyphenolic compounds. *Adv Exp Med Biol.,* 1986, vol. 199, 509-16
- **Carmona A.** Tannins: thermostable pigments which complex dietary proteins and inhibit digestive enzymes. *Arch Latinoam Nutr.,* Décembre 1996, vol. 44 (4), 31S-35S
- **Tebib K ; Rouanet JM ; Besancon P.** Effect of grape seed tannins on the activity of some rat intestinal enzyme activities. *Enzyme Protein,* 1994, vol. 48 (1), 51-60
- **Auger C ; Gerain P ; Laurent-Bichon F ; Portet K ; Bomet A ; Caporiccio B ; Cros G ; Teissedre PL ; Rouanet JM.** Phenolics from commercialized grape extracts prevent early atherosclerotic lesions in hamsters by mechanisms other than antioxidant effect. *J Agric Food Chem.,* 11 Août 2004, vol. 52 (16), 5297-302
- **Kurowska EM ; Manthey JA.** Hypolipidemic effects and absorption of citrus polymethoxylated flavones in hamsters with diet-induced hypercholesterolemia. *J Agric Food Chem.,* 19 Mai 2004, vol. 52 (10), 2879-86
- **Wiseman SA ; Tijburg LB ; van de Put FH.** Olive oil phenolics protect LDL and spare vitamin E in the hamster. *Lipids,* Novembre 2002, vol. 37 (11), 1053-7
- **Gilat T ; Leikin-Frenkel A ; Goldiner I ; Juhel C ; Lafont H ; Gobbi D ; Konikoff FM.** Prevention of diet-induced fatty liver in experimental animals by the oral administration of a fatty acid bile acid conjugate (FABAC). *Hepatology,* Août 2003, vol. 38 (2), 436-42
- **Nicolosi RJ ; Wilson TA ; Rogers EJ ; Kritchevsky D.** Effects of specific fatty acids (8:0, 14:0, cis-18:1, trans-18:1) on plasma lipoproteins, early atherogenic potential, and LDL oxidative properties in the hamster. *J Lipid Res.,* Octobre 1998, vol. 39 (10), 1972-80